# EUROPEAN PATENT SPECIFICATION

(11) **EP 0 449 663 B1**
(45) Date of publication and mention of the grant of the patent: **19.06.1996**
(21) Application number: 91302837.9
(22) Date of filing: 28.03.1991
(51) Int. Cl.: A61B 17/02

(54) **Abdominal cavity organ retractor**
Retraktor für Organe der Bauchhöhle
Rétracteur pour les organes de la cavité abdominale

(30) Priority: 29.03.1990 US 501241
(43) Date of publication of application: 02.10.1991
(73) Proprietor: United States Surgical Corporation, Norwalk, Connecticut 06856 (US)
(72) Inventor: Bailey, Robert W., Reisterstown, MD 21136 (US)
(74) Representative: Marsh, Roy David

(56) References cited:
- EP-A- 0 246 086
- DE-A- 3 709 706
- FR-A- 2 082 987
- US-A- 3 837 345
- US-A- 4 459 978

## Description

This invention relates to the field of surgery, and in particular to devices which are known in the art as retractors.

A surgeon, when performing an operation on a patient, is often obstructed in his efforts to excise diseased or damaged tissues or organs by surrounding tissues, fatty deposits, arteries, or other organs. It has generally been the case, when performing gastro intestinal surgery, i.e. surgery within the abdominal cavity, to make a large cut in the abdomen wall to produce a suitable opening to allow access to the interior organs. This cut was generally large enough to allow the use of human hands, either the surgeons or those of a member of the surgical team, as a retractor. Surgical personnel would thus insert their hands through the incision into the abdominal cavity to push and hold organs and other obstructing components away from the surgical objective.

Recently, as a result of the evolution in electronic video technology, a surgical procedure known as laparoscopic surgery has undergone a marked increase in popularity. The laparoscope consists of a long thin rigid tube. Residing at one end of the tube is a viewing lens; at the other is a camera hook up and an eye piece. A small incision in the area of the surgical objective is made and the laparoscope is partially inserted into this incision, viewing lens first. High definition video cameras and monitors are then attached to the camera hook up joint on the part of the laparoscope which remains on the exterior. In this manner, a surgical team can get a clear picture of the affected internal area without resorting to radical, disfiguring surgical incisions to physically open the patient. Numerous other small puncture wounds are then made through the surface of the skin also in the vicinity of the surgical objective. Through these incisions, miniaturized surgical instruments such as scissors, forceps, clamps and scalpels are insertable to perform the surgical procedure. The entire interior procedure is monitored from the exterior through the high definition television monitor. In this manner radical incisions and scarring are avoided while undertaking surgical repair or removal of damaged or diseased organs. Another benefit of laparoscopic surgery is the significantly reduced recovery time, when compared to standard surgical procedures, due to the minuscule size of the scalpel wounds and avoidance of the massive internal traumatization known in standard surgical procedures. Accompanying the reduced recovery time are, of course, greatly reduced costs.

Until recently, laparoscopic surgery was limited to gynecological and arthroscopic procedures. Recently, however, innovative surgeons have been using the procedure to perform cholecystectomy, commonly known as gallbladder removal. A problem encountered by surgeons when performing laparoscopic cholecystectomy is the intrudence of other internal abdominal organs into the surgical objective area i.e. gallbladder area. Abdominal organs such as the stomach, intestine and liver are known to be floppy and to overlap one another. Standard gallbladder surgery of the past, in which a huge incision was made in the patient's abdominal wall, allowed for the insertion of hands into the patient's abdominal cavity to push and hold these floppy organs away from the gallbladder while the surgeon effected the necessary removal. Laparoscopic cholecystectomy, with its minute surgical incisions, does not allow for the insertion of a human hand into the cavity to retract the floppy organs.

There is a need therefore to provide an instrument which can be inserted through a tiny surgical incision in the abdominal wall, to be used during laparoscopic surgery within the abdominal cavity, to push and hold neighboring internal organs away from that organ which is being excised or repaired. For example, during the known procedure of laparoscopic cholecystectomy, a retractor instrument is needed to push and hold neighboring organs away from the diseased gallbladder.

It is a general object of this invention to provide a retractor device to be used during laparoscopic surgical procedures.

It is an object of this invention to provide a retractor device to be used during laparoscopic surgery within the abdominal cavity.

Another object of the invention is to provide a long thin tube-like instrument suitable for insertion into the abdomen through a tiny incision in the abdomen wall to be used when performing laparoscopic abdominal surgery.

It is a further object of this invention to provide pivotable finger-like protrusions extending from that end of the tube which is inserted into the abdomen to be used as a push and hold means and for establishing means of communication to these protuberances from levers that depend from that end of the tube which remains exterior of the abdomen, said levers to be used in controlling the workings of the push and hold means within the abdomen.

SU-736949 discloses a shaft tool with a shovel blade defined by three co-planar flat blades which lie in the same plane as the shaft. The pre-characterising part of claim 1 below is framed with reference to the retractor with flexible fingers which is disclosed in DE-A-3709706. The present invention is defined by the characterising features of the claim.

In one embodiment of the invention a long narrow tube-like structure is provided. The tube is generally hollow and open at each end. At one end a plurality of finger-like protuberances are positioned within the tube on the same axial plane as the tube and will extend outwardly from the tube upon movement of the control means at the other end, such as a pair of lever means which depend from the opening. Within the narrow elongated body, comprising a tube, reside the internal mechanism and means for communicating movement of the levers to movement of the finger-like protuberances. In one example the levers are biased apart and manipulation of the levers so as to bring them close together is communicated to the finger-like protuberances so as to spread them out in a fan type pattern. A release of force on the levers allow the levers to return to their normally apart position. This, in turn, allows the finger-like protuberances to return to their original position, i.e. close together on the same axial plane as the narrow elongated body structure. In this manner the retractor can be inserted into the abdominal cavity, through an incision in the abdominal wall or through a sheath which has been placed through an incision in the abdominal wall, and guided to the surgical objective by monitoring the movement of the retractor within the cavity on the laparoscope and its associated high definition television monitor. Once the retractor has been positioned suitably near the organ of interest, the surgeon or a member of the surgical team, by squeezing together the levers depending from that end of the retractor which has not been inserted into the cavity, can cause the finger-like protuberances to spread apart in a fan-like pattern to push and hold surrounding organs away from that organ which is the surgical objective. In this manner abdominal laparoscopic surgery is greatly simplified as the problem caused by floppy, overlapping organs encroaching upon the organ which is the surgical objective has been eliminated.

The above-listed preferred aspects are not intended as limiting. For instance, the device can be constructed with the finger-like protrusions nominally in the opened up fan-like pattern. In this manner, forcing the levers together will cause the protrusions to come together on the same axial plane as the elongated body. The finger-like protuberances can then be inserted into the abdominal cavity while maintaining force on the levers to hold them together. Once near the surgical objective, the levers can be released causing the finger-like protuberances to open up into their normally open fan-like pattern pushing and holding intruding organs out of the way of the surgeon. When used in this manner, the requirement of having a member of the surgical team tend to the retractor levers and hold them together is thus eliminated.

It is contemplated for this embodiment that the lever device for activating the retractor finger-like protrusions be replaced by a syringe-like mechanism in which a pair of finger grips are provided which radially extend from and are secured to the tubular body. A thumb activated push button is attached to the inner rod to control the extension of the finger-like protrusions. The finger grips may be rotatable about the tubular body so that position of the protrusions along the common diametric line in relation to the finger grips may be oriented to suit the needs of the surgical team for that particular operation. Preferably, the diametric line of the protrusions is parallel to the diametric positioning line of the finger grips until altered by the surgical team.

It is also possible to manufacture the device with the levers depending straight out from the elongated body on the same axial plane or have them depend away from the body at some angle, for instance at a 90° angle to the retractor body. It will also be possible to form finger loops in the levers for insertion of the fingers of the member of the surgical team operating the retractor during the laparoscopic surgery. The elongated body structure of the retractor can also be made to be flexible so as to facilitate the positioning of the protruding fingers in close proximity to the area of the surgical objective.

In the accompanying drawings of embodiments of the invention:
Fig. 1 illustrates the retractor having a slide button control with the engagement members in a closed state;
Fig. 2 illustrates the retractor having a slide button control shown with the engagement members in a partially opened state;
Fig. 3 illustrates the retractor with lever controls and the engagement members in a closed state;
Fig. 4. illustrates an alternate embodiment of Fig. 3 in which the engagement members are in a normally opened state;
Fig. 5 illustrates yet another embodiment of Fig. 3 with handle controls;
Fig. 6 illustrates the alternate sheath embodiment of the retractor showing the engagement members withdrawn into the sheath;
Fig. 7 illustrates the retractor of Fig. 6 showing the head depressed and the engagement members outside of the sheath and opened;
Fig. 8. illustrates a sheath embodiment of the retractor in which levers are used to exert force on the head;
Fig. 9 illustrates an alternate embodiment of the device shown in Fig. 8 in whch the secondary lever is slidably attached to the head;
Fig. 10 illustrates another sheath embodiment of the retractor in which the engagement members normally reside without the sheath in an open position and lever controls are used to close and withdraw the engagement members within the sheath;
Fig. 11 illustrates a sheath embodiment of the invention in which fixed handles are used to force out the engagement members from within the sheath;
Fig. 12 illustrates a detailed view showing one method of biasing the engagement members open;
Fig. 13 illustrates a cut-away view of the operation of the engagement members of the embodiments portrayed in Figs. 1, 2, 3 and 5;
Fig. 14 illustrates a cut-away view of the operation of the engagement members of the embodiments portrayed in Fig. 4; and
Fig. 15 illustrates the retractor having an electromechanical control.

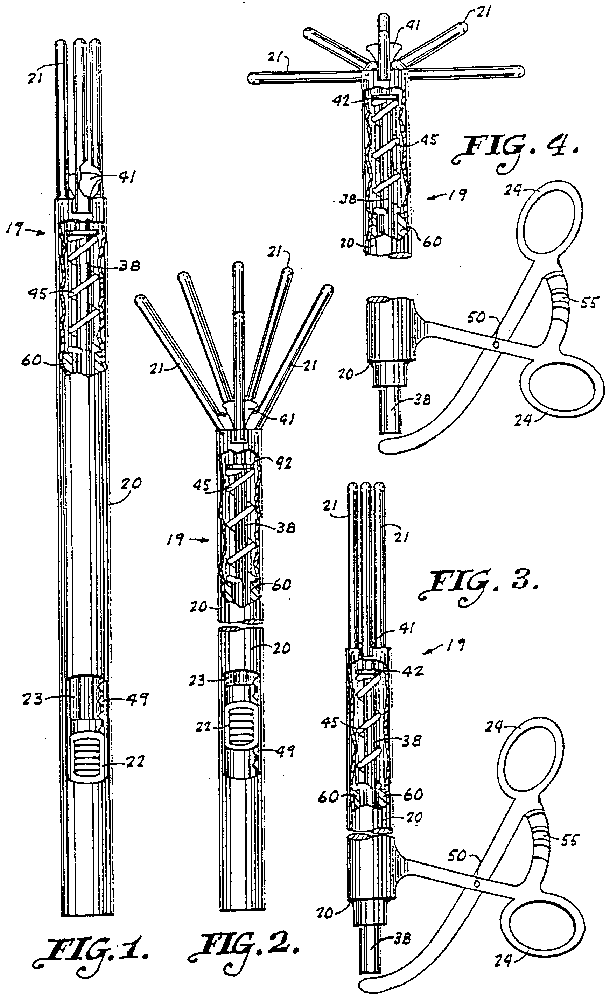

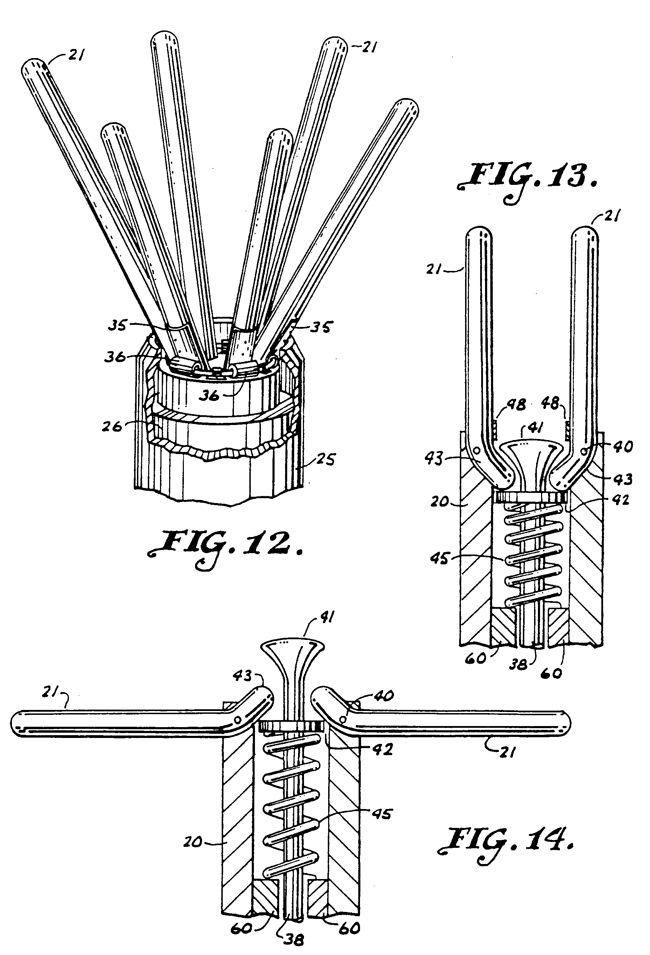

Referring to Figs. 1 and 13, the retractor 19 is shown with engagement members 21 biased to a closed resting position and control button 22 biased to a set position within slide path 23. In this manner, engagement members 21 and a portion of tube-like body 20 are insertable through a small incision in an abdominal wall. Control button 22 can then be forcibly slid within slide path 23 to effect an opening of engagement members 21 into a fanned-out pattern as shown in Fig. 2. In this manner internal organs can be pushed and held away from that organ which is the surgical objective.

Fig. 13 more clearly depicts the operation of the engagement members 21. The button 22 is fixedly connected to movable shaft 38. Engagement members 21 are pivotally connected to tube-like body 20 by pivot joints 40, so that the outward movement of the engagement members 21 occurs due to pivoting of the members 21 rather than by outward deflection of flexible members, as in the prior art. Movable shaft 38 terminates with knob 41. Disc 42 is fixedly attached to movable shaft 38 at a point just below knob 41. The engagement members 21 have lips 43 that are enclosed between knob 41 and disc 41. Spring 45 circumferentially encompasses movable shaft 38 and is fixedly attached at one end to disc 42. The other end of spring 45 is fixedly attached to inner guide wall 60. Inner guide wall 60 can run any distance through tube-like body 20 but must terminate a sufficient distance below disc 42 to enable the placement of spring 45 between the top of inner guide wall 60 and disc 42.

Operation of engagement members 21 is effected by sliding button 22 which produces a movement of movable shaft 38 towards the engagement members 21. Disc 42 is forced against the pull of spring 45. Disc 42 pushes against lips 43 causing engagement members 21 to rotate about pivots 40 and assume an open position. It can be seen that the farther the movable shaft 38 is moved toward engagement members 21 the greater the rotation of the engagement members 21 about pivots 40, and hence the greater diameter of the open, fanned-out pattern. A cessation of force on control button 22 allows control button 22 to return to its original set position and engagement members 21 to return to their normally closed resting position as shown in Fig. 2. This action is achieved due to the pull of spring 45 upon disc 42, which in the absence of any counter forces, pulls movable shaft 38, disc 42 and knob 41 in a direction opposite engagement members 21. Knob 41 will be forced against lips 43 which will cause reverse rotation of engagement members 21 about pivots 40 to bring engagement members 21 to a closed position in which they occupy the same axial plane as the elongated tube-like body 20. Stop pieces 48 may be placed in the position shown in Fig. 13 to prevent excessive inward rotation of engagement members 21. Stop pieces 48 may alternatively be placed under lips 43.

It is also possible, but not shown in the drawings, to fixedly attach at least one engagement member to knob 41. A structure of this sort would be beneficial in terms of operation of the device since these fixedly attached engagement members would aid in displacement of organs and prevent a displaced organ from jumping over one of the movable engagement members 21 and occupying its position before displacement.

Engagement members 21 can be of any shape as long as their retracting function can be achieved. Preferably engagement members 21 are of cylindrical shape with rounded exposed ends so that soft tissue injury is minimized.

It is also preferred that engagement members 21 are constructed of a substantially rigid material which means that any bending or deflecting of the engagement members 21 in the open or fanned-out position is limited. While some limited deflection may help to avoid damage to the organs, the material of which engagement members 21 nevertheless must be substantially rigid, that is to say, rigid enough to properly hold the organs in the displaced location. As described above, the fan-like arrangement of engagement members 21, in the extended position, is effected by a pivoting action of members 21 about pivot points 40 as required by the rigid nature of the members, as opposed to the fanning out of the members as in the prior art due to the spring-like flexibility of the prior art engagement members.

Referring to Figs 1 and 2, a means to hold or lock the activating button 22 at points within slide path 23 are preferred embodiments and allow for controlling the diameter of the fan-out pattern of engagement members 21 and also for hands-off utilization of the retractor 19. The holding means could be, for example, notches 49 along slide path 23 for physically holding control button 22 at points through its path of travel through slide path 23.

A modification of this embodiment of the device is shown in Figure 3. Levers 24 are used instead of a button to force movement of movable shaft 38. Levers 24 are pivotally joined at pin 50 with one of the levers making contact with the end of movable shaft 38 opposite engagement members 21. As seen in Figure 3, movable shaft 38 is shown in this embodiment to extend outward from tube-like body 20. Operation of engagement members 21 upon forcing of movable shaft in the direction of engagement members 21 by squeezing of levers 24 is identical to that previously described when referencing Fig. 13 and such description is incorporated by reference herein.

Referring to Figures 4 and 14, another embodiment using levers 24 is shown. In this embodiment engagement members 21 are normally open. The normally open state results from spring 45, which is affixed at the top of the fixed inner guide wall 60 and disc 42, exerting a push force against disc 42 in the absence of any other forces. Disc 42, which is fixedly attached to movable shaft 38 impacts upon lips 43 to cause outward rotation of engagement members 21 about pivots 40. In order to close engagement members 21, it is necessary to squeeze levers 24 together. This pulls movable shaft 38 back towards the end of the device opposite engagement members 21 due to the connection with one of levers 24 with movable shaft 38 at connection 51. Drawing movable shaft 38 back towards levers 24 causes knob 41 to impact upon lips 43 while compressing spring 45 to force rotation of engagement members 21 about pivots 40 in a direction that will cause engagement members 21 to close together along the same axial plane as tube-like body 20. Releasing force on levers 24 causes spring 45 to expand which in turn presses disc 42 on the underside of lips 43 causing engagement members 21 to rotate outward into their normally opened, fanned-out state.

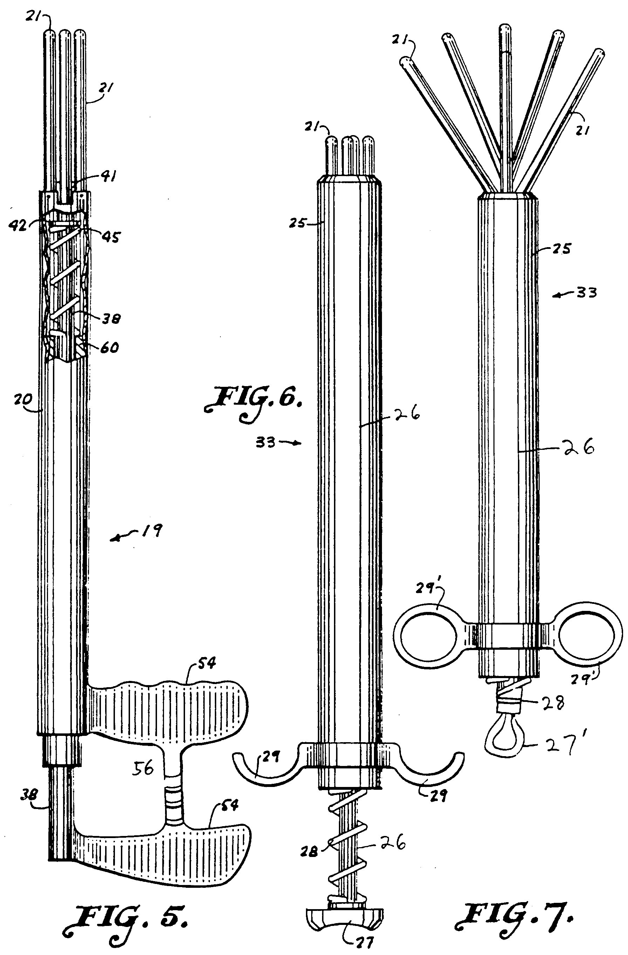

Another embodiment of the device is shown in Fig. 5 in which handles 54, one attached to the elongated tube-like body 20 at the end away from engagement members 21, the other attached to the end of movable shaft 38 at the end opposite engagement members 21, are utilized for control. By gripping and squeezing handles 54, movable shaft 38 is moved in the direction towards engagement members 21 causing an opening of engagement members 21 in the manner previously described when referring to Fig. 13.

In any of the aforementioned embodiments which use levers 24 or handles 54, it is preferable to include means to hold the levers 24 or handles 54 at various points in their movement towards one another. This could be accomplished, for example, by an adjustable clip 55 to hold the levers 24 or handles 54 together, or by equipping each lever 24 or handle 54 with a horizontal appendage 56 with backwards facing teeth for interlocking the levers 24 or handles 54.

Referring to Figure 6, sheath 25 is shown. Residing within sheath 25 is rod 26. Rod 26 is slidably mounted within sheath 25 so that force on rod head 27 against bias force propels the rod head 27 towards sheath 25 allowing engagement members 21 to exit from within sheath 25. The absence of the containing action caused by sheath 25 on engagement members 21 when engagement members 21 are without sheath 25 allows engagement members 21 to expand into an opened-up fanned-out pattern. This occurrence is demonstrated by Fig. 7. In this instance the bias is shown to be a spring 28 wrapped circumferentially around rod 26 just below rod head 27′ , shown in this instance as a loop, and exterior to sheath 25. This is not limiting as the spring could conceivably be located within the sheath or some other type of bias could be used. Also shown by Figs. 6 and 7 are flange members 29 and 29′ in the form of partial or complete loops mounted on the exterior of sheath 25. Flange members 29 and 29′ facilitate operation of the device 33 by providing the user with means to grasp the device 33 such as with the middle finger and ring finger while applying force, such as with the thumb, to rod head 27 or 27′ to force exit and expansion of engagement members 21.

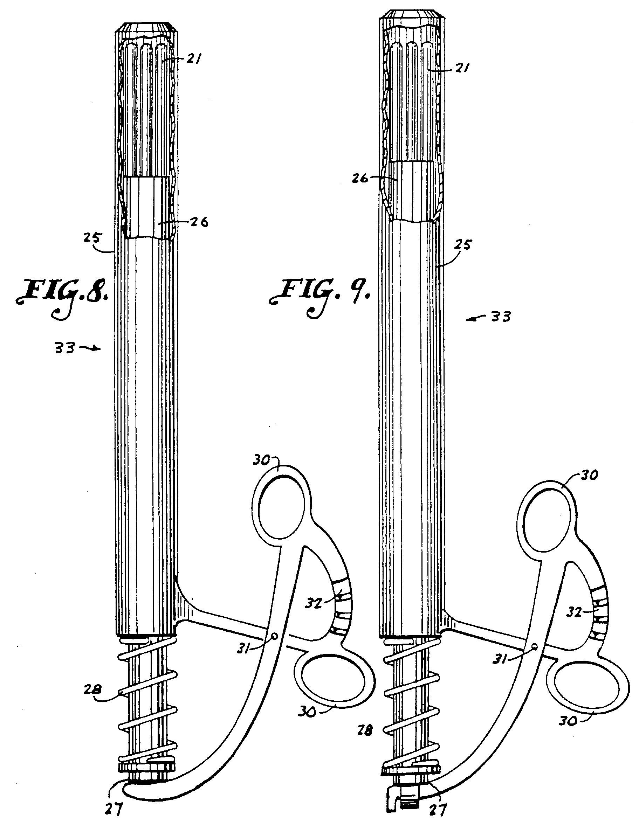

Figs. 8 and 9 are alternate embodiments of the sheath type mechanism disclosed in Figs. 6 and 7 which further provide levers 30 for exerting the necessary force on rod head 27 to effectuate action of engagement members 21. In the embodiment as shown in Fig. 8 one lever is fixedly attached to sheath 25 while the other lever imparts force on rod head 27 but is not physically attached to rod head 27. Fig. 9 shows the same retractor 33 but with a lever 30 slidably attached to rod head 27. In each shown embodiment of the retractor 33 with levers 30, the levers 30 are connected to one another at pin 31. Biasing means is shown in both figures to be accomplished by use of spring 28 located circumferentially on rod 25 adjacent to rod head 27 and exterior to sheath 25. A means to hold the levers 30 in numerous closed or partially closed positions to fixedly secure the rod 26 within the sheath 25 at numerous positions to effect the fan out diameter of engagement members 21 is shown as toothed clip 32.

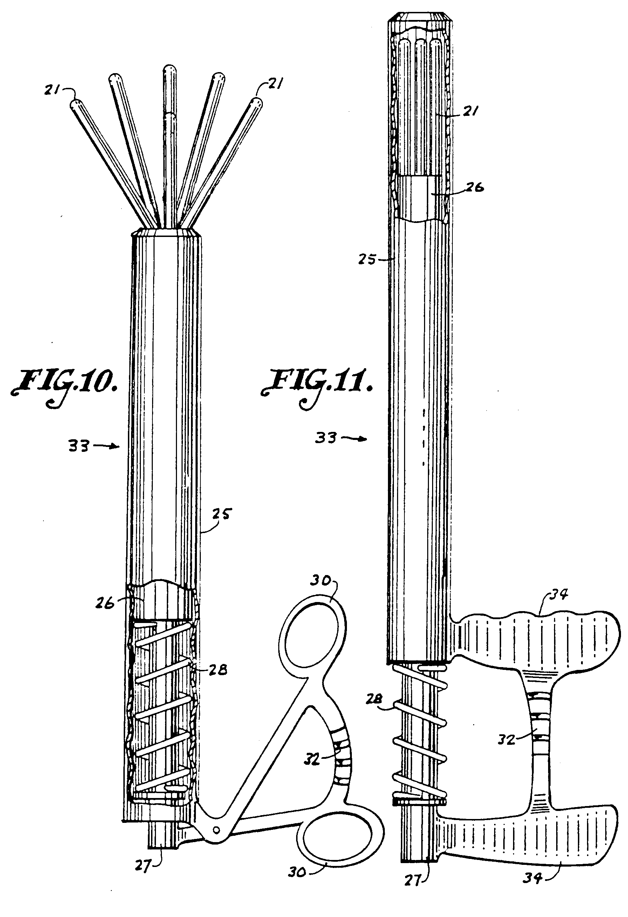

Figure 10 is an alternate embodiment of the sheath type retractor 33 wherein the normal position, i.e. with no forces being exerted, of the components of the retractor 33 are as shown. The rod head 27 is biased, for example, by spring 28 to a position against sheath 25 so that engagement members 21 normally reside without sheath 25 and display an opened fanned out pattern. Force exerted on levers 30 in the form of squeezing the bottom of the levers 30 together acts to pull rod head 27 away from sheath 25 forcing engagement members 21 into a closed position as they are withdrawn within sheath 25. As with any of the other embodiments of the retractor various bias means instead of spring 28 can be utilized as well as varying means to hold rod 26 within sheath 25 at different lateral locations to control the diameter of the spread of engagement members 21.

Fig. 11 is yet another embodiment of the sheath type embodiment of retractor 33 in which handles 34 control engagement members 21. For proper use of the device as shown in Fig. 11, the user can grasp the handles 34 and squeeze them together against the force of bias which in this instance is shown to be spring 28, to force rod 26 towards the open end of sheath 25 so that engagement members 21 can exit from within sheath 25 and assume an open fanned-out pattern.

Fig. 12 is a close-up view of one of the mechanisms which may be utilized to effectuate an opening of engagement members 21 when they are forced out of sheath 25. Engagement members 21 are shown to be attached to the end of rod 26 by pivot joints 36. When forced without the confining boundaries of sheath 25, spring fan outs 35 pull back on engagement members 21 to pivot them into an opened, fanned-out pattern. As can be seen, the amount by which engagement members 21 are forced without sheath 25 will directly affect the diameter of the fanned-out pattern caused by spring fan outs 35 acting in conjunction with pivot joints 36.

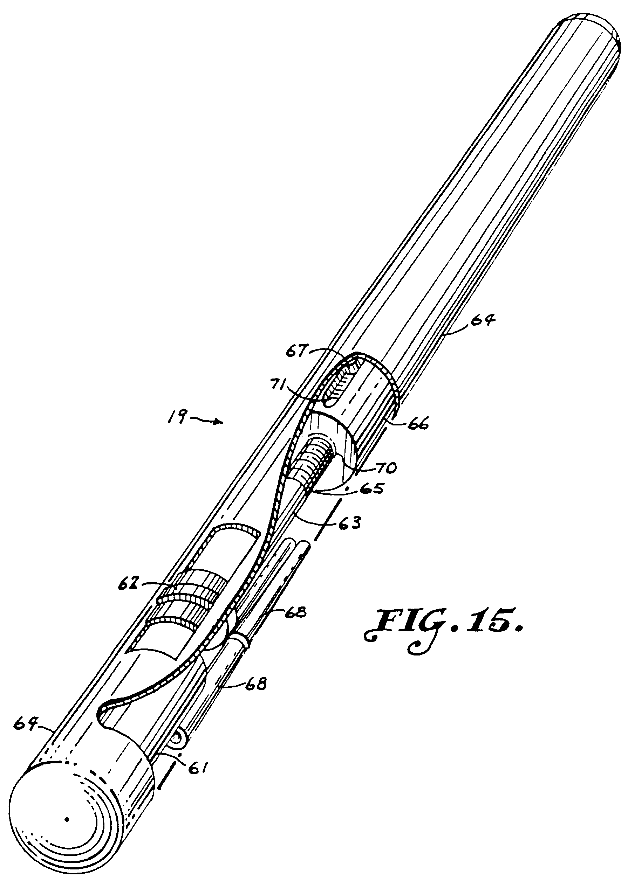

Recognizing that lateral displacement of a movable shaft or rod is essential for operation of any embodiment of the device, it is possible to utilize an electromechanical device, such as a motor, to achieve such lateral displacement. Fig. 15 is one example of how a motor might be used to produce lateral displacement of a shaft or rod.

Within retractor 19 and casing 64 is found reversible motor 61. Reversible motors of this design are known in the art and require no further explanation. Reversible motor 61 is shown to be controlled by slide switch 62. Slide switch 62 can be of the known type having a center-off position. Switch 62 can be displaced in either direction above or below the center-off position.

Displacement of switch 62 in one direction causes rotation of threaded shaft 63 in one rotational direction; displacement of switch 62 in the other direction causes opposite rotation of threaded shift 63.

Threaded shaft 63, has threads 65 which engage threads 70 of hollow cylinder 66. Rotation of threaded shaft 63 laterally displaces hollow cylinder 66 due to the engagement of threads 65 and 70. It can be seen that lateral displacement of hollow cylinder 66 can occur in either direction resulting from the ability to reverse the rotational direction of threaded shaft 63.

In order to prevent rotation of hollow cylinder 66, groove 71 is cut through the wall of hollow cylinder 66. Pegs 67 are fixedly attached to the inner wall of casing 64 and protrude inward through groove 71. In this manner, pegs 67 prevent rotation of hollow cylinder 66 while allowing lateral displacement of hollow cylinder 66.

The retractor 19 shown and described in Fig. 15 is preferably a portable self-contained unit. In furtherance of this objective, reversible motor 61 receives electrical power from on-board batteries 68.

The control means disclosed in Fig. 15 would suitably replace any of the control mechanisms disclosed in Figs. 1-11. The control means of Fig. 15 would control engagement members 21 by virtue of lateral displacement of hollow cylinder 66 by the methods as shown and described in Figs. 12-14. With respect to Figs. 13 and 14, spring 45 would not be necessary and could be eliminated when the control mechanism shown in Fig. 15 and described above is utilized. Various methods of positioning the rod within the sheath or, relatedly, holding the lever means in various positions with respect to one another, are possible and will be evident to those skilled in the art.

## Claims

1. A laparoscopic retractor device (19) for displacing tissue and organs within an abdominal cavity, comprising:
an elongate tubular body member (20) having an opening at a first end,
elongate rod means (38) positioned coaxially within said body member,
a plurality of finger members (21) operatively connected to said rod means (38) at a first end of said rod means adjacent said first end of said body member (20), for spreading radially outwards around the long axis of the tubular body, whereby said tissue or organ is receivable within a pushing circle defined by the tips of the spread fingers, in order that a force applied on the said long axis, through the tubular body and directed from the second end towards the first end can be applied to push said tissue or organ away from the user of the device,
an activating mechanism positioned at a second end of said body member and said rod means and cooperatingly engaged with said body member and said rod means to effect movement of said finger members,
wherein movement of said activating mechanism from a rest position in a first direction to a set position deploys said finger members from a closed disposition to a deployed disposition, outside said body member, in which said finger members are arranged in a radially spread pattern, and further wherein reverse movement of said activating mechanism from said set position in a second direction to said rest position retracts said finger members to lie parallel with the rod means and tubular body in said closed disposition;
characterised in that:
i) the finger members are substantially rigid and straight and, in the closed disposition, parallel to the long axis of the tubular body member (20);
ii) the finger members are actuated by the rod means by a pivotal connection; and
iii) the movement of the fingers from the closed to the deployed disposition extends to an extreme deployed disposition in which the entire length of each of the straight fingers lies at an angle to the said long axis.

2. A retractor device according to claim 1, wherein said finger members are rigid and cylindrically shaped, said finger members terminating in a rounded spherical end.

3. A retractor device according to claim 1 or 2, wherein said rod means comprises a rod member (38) and said activating mechanism is adapted to effect reciprocating movement of said rod member within said body member (20).

4. A retractor device according to claim 3, wherein movement of said activating mechanism in said first direction moves said rod member through said body member towards said first end, and movement of said activating mechanism in said second direction moves said rod member into said body member.

5. A retractor device according to any one of the preceding claims, wherein said finger members each have a radially inner end which is accommodated between a knob at the first end of the rod means (38) and a disc (42) near the first end.

6. A retractor device according to any one of the preceding claims, whrein said rod member is biased towards said rest position by a spring (45).

7. A retractor device according to any one of the preceding claims, wherein said activating mechanism comprises a pair of scissor-like handle members (24), a first handle member being connected to said body member and a second handle member being connected to said rod member.

8. A retractor device according to any one of claims 1 to 6, wherein said activating mechanism comprises a pair of finger grips (90) attached to said body member and a push button (92) attached to an end of said rod member.

9. A retractor device according to claim 8, wherein said finger grips (90) are rotatable about said body member.

## Patentansprüche

1. Laparoskopisches Retraktorinstrument (19) zum Verlagern von Gewebe und Organen innerhalb einer Bauchhöhle, umfassend:
ein langgestrecktes röhrenförmiges Körperelement (20) mit einer Öffnung an einem ersten Ende,
eine koaxial innerhalb des besagten Körperelements angeordnete langgestreckte Stangeneinrichtung (38),
eine Mehrzahl von Fingerelementen (21), die zum Aufspreizen in radialer Richtung nach außen um die lange Achse des röhrenförmigen Körpers herum an einem ersten Ende der besagten Stangeneinrichtung angrenzend an das besagte erste Ende des besagten Körperelements (20) funktionell mit der besagten Stangeneinrichtung (38) verbunden sind, wodurch das besagte Gewebe oder Organ innerhalb eines von den spitzen der aufgespreizten Finger begrenzten Schiebekreises aufnehmbar ist, damit eine durch den röhrenförmigen Körper auf die besagte lange Achse aufgebrachte und vom zweiten Ende in Richtung des ersten Endes gerichtete Kraft aufgebracht werden kann, um das besagte Gewebe oder Organ vom Benutzer des Instruments weg zu drücken,
einen Aktivierungsmechanismus, der an einem zweiten Ende des besagten Körperelements und der besagten Stangeneinrichtung angeordnet ist und unter Zusammenwirken mit dem besagten Körperelement und der besagten Stangeneinrichtung im Eingriff steht, um eine Bewegung der besagten Fingerelemente zu bewirken,
wobei eine Bewegung des besagten Aktivierungsmechanismus aus einer Ruheposition in einer ersten Richtung in eine Einstellposition die besagten Fingerelemente aus einer geschlossenen Anordnung in eine ausgebrachte Anordnung außerhalb des besagten Körperelements ausbringt, in welcher die besagten Fingerelemente in einem in radialer Richtung aufgespreizten Muster angeordnet sind, und wobei weiter eine umgekehrte Bewegung des besagten Aktivierungsmechanismus aus der besagten Einstellposition in einer zweiten Richtung in die besagte Ruheposition die besagten Fingerelemente zurückzieht, so daß sie in der besagten geschlossenen Anordnung parallel zur Stangeneinrichtung und zum röhrenförmigen Körper liegen;
dadurch gekennzeichnet, daß:
i) die Fingerelemente im wesentlichen starr und gerade und in der geschlossenen Anordnung parallel zur langen Achse des röhrenförmigen Körperelements (20) sind;
ii) die Fingerelemente mittels einer Schwenkverbindung von der Stangeneinrichtung betätigt werden; und
iii) die Bewegung der Finger aus der geschlossenen in die ausgebrachte Anordnung sich bis zu einer extremen ausgebrachten Anordnung erstreckt, in welcher die gesamte Länge von jedem der geraden Finger unter einem Winkel zu der besagten langen Achse angeordnet ist.

2. Retraktorinstrument nach Anspruch 1, dadurch gekennzeichnet, daß die besagten Fingerelemente starr und zylindrisch geformt sind, wobei die besagten Fingerelemente in einem gerundeten kugeligen Ende enden.

3. Retraktorinstrument nach Anspruch 1 oder 2, dadurch gekennzeichnet, daß die besagte Stangeneinrichtung ein Stangenelement (38) umfaßt, und der besagte Aktivierungsmechanismus so angepaßt ist, daß er eine Hin- und Herbewegung des besagten Stangenelements innerhalb des besagten Körperelements (20) bewirkt.

4. Retraktorinstrument nach Anspruch 3, dadurch gekennzeichnet, daß eine Bewegung des besagten Aktivierungsmechanismus in der besagten ersten Richtung das besagte Stangenelement durch das besagte Körperelement in Richtung des besagten ersten Endes bewegt, und eine Bewegung des besagten Aktivierungsmechanismus in der besagten zweiten Richtung das besagte Stangenelement in das besagte Körperelement bewegt.

5. Retraktorinstrument nach einem beliebigen der vorangehenden Ansprüche, dadurch gekennzeichnet, daß die besagten Fingerelemente jeweils ein radial inneres Ende aufweisen, welches zwischen einem Knopf am ersten Ende der Stangeneinrichtung (38) und einer Scheibe (42) nahe dem ersten Ende untergebracht ist.

6. Retraktorinstrument nach einem beliebigen der vorangehenden Ansprüche, dadurch gekennzeichnet, daß das besagte Stangenelement durch eine Feder (45) in Richtung der besagten Ruheposition vorgespannt ist.

7. Retraktorinstrument nach einem beliebigen der vorangehenden Ansprüche, dadurch gekennzeichnet, daß der besagte Aktivierungsmechanismus ein Paar scherenartige Griffelemente (24) umfaßt, wobei ein erstes Griffelement mit dem besagten Körperelement verbunden ist, und ein zweites Griffelement mit dem besagten Stangenelement verbunden ist.

8. Retraktorinstrument nach einem beliebigen der Ansprüche 1 bis 6, dadurch gekennzeichnet, daß der besagte Aktivierungsmechanismus ein Paar an dem besagten Körperelement angebrachte Fingergriffe (90) und einen an einem Ende des besagten Stangenelements angebrachten Schiebeknopf (92) umfaßt.

9. Retraktorinstrument nach Anspruch 8, dadurch gekennzeichnet, daß die besagten Fingergriffe (90) um das besagte Körperelement drehbar sind.

## Revendications

1. Dispositif rétracteur laparoscopique (19) pour déplacer le tissu et les organes dans une cavité abdominale, comprenant :
un corps tubulaire allongé (20) ayant une ouverture à une première extrémité,
un moyen formant tige allongée (38) placé coaxialement dans le corps,
un certain nombre de doigts (21) activement connectés audit moyen formant tige (38) à une première extrémité dudit moyen formant tige à proximité de ladite première extrémité dudit corps (20), pour s'écarter radialement vers l'extérieur autour de l'axe long du corps tubulaire, ainsi ledit tissu ou organe peut être reçu dans un cercle de poussée défini par les extrémités des doigts afin qu'une force appliquée sur ledit axe long, à travers le corps tubulaire et dirigé à partir de la seconde extrémité vers la première extrémité, puisse être appliquée pour pousser ledit tissu ou organe loin de l'utilisateur du dispositif,
un mécanisme d'activation placé à une seconde extrémité dudit corps et dudit moyen formant tige et en engagement coopératif avec ledit corps et ledit moyen formant tige pour effectuer le mouvement desdits doigts,
où le mouvement dudit mécanisme d'activation d'une position de repos dans une première direction à une position de consigne déploie lesdits doigts à partir d'une disposition fermée jusqu'à une position déployée, en dehors dudit corps, où lesdits doigts sont agencés en un motif radialement étendu, et de plus où le mouvement inverse dudit mécanisme d'activation à partir de ladite position de consigne dans une seconde direction jusqu'à ladite position de repos, retire lesdits doigts pour qu'ils se trouvent parallèles à ladite tige et au corps tubulaire dans ladite disposition fermée ;
caractérisé en ce que :
i) les doigts sont sensiblement rigides et droits et, à la disposition fermée, ils sont parallèles à l'axe long du corps tubulaire (20) ;
ii) les doigts sont actionnés par le moyen formant tige par une connexion pivotante ; et
iii) le mouvement des doigts de la disposition fermée à celle déployée s'étend jusqu'à une disposition extrême déployée dans laquelle toute la longueur de chacun des doigts droits se trouve à un angle par rapport audit axe long.

2. Dispositif rétracteur selon la revendication 1, où lesdits doigts sont rigides et de forme cylindrique, lesdits doigts se terminant par une extrémité sphérique arrondie.

3. Dispositif rétracteur selon la revendication 1 ou 2, où ledit moyen formant tige comprend une tige (38) et ledit mécanisme d'activation est adapté à effectuer le mouvement alternatif de ladite tige dans ledit corps (20).

4. Dispositif rétracteur selon la revendication 3, où le mouvement dudit mécanisme d'activation dans ladite première direction déplace ladite tige à travers ledit corps vers ladite première extrémité et le mouvement dudit mécanisme d'activation dans ladite seconde direction déplace ladite tige dans ledit corps.

5. Dispositif rétracteur selon l'une quelconque des revendications précédentes, où chacun desdits doigts a une extrémité radialement interne qui est reçue entre un bouton à la première extrémité de la tige (38) et un disque (42) à proximité de la première extrémité.

6. Dispositif rétracteur selon l'une quelconque des revendications précédentes, où ladite tige est sollicitée vers ladite position de repos par un ressort (45).

7. Dispositif rétracteur selon l'une quelconque des revendications précédentes, où ledit mécanisme d'activation comprend une paire de poignées (24) semblables à des ciseaux, une première poignée étant connectée audit corps et une seconde poignée étant connectée à ladite tige.

8. Dispositif rétracteur selon l'une quelconque des revendications 1 à 6, où ledit mécanisme d'activation comprend deux pinces à doigts (90) attachées audit corps et un bouton poussoir (92) attaché à une extrémité de ladite tige.

9. Dispositif rétracteur selon la revendication 8, où lesdites pinces à doigts (90) sont rotatives autour dudit corps.
